# EUROPEAN PATENT APPLICATION

(11) **EP 3 992 202 A1**
(43) Date of publication of application: **04.05.2022**
(21) Application number: 20784232.9
(22) Date of filing: 06.04.2020
(51) Int. Cl.: C07K 7/08, A61K 38/10, A61P 29/00, A61P 37/02, A61P 37/08

(54) **SYNTHETIC PEPTIDES WITH AFFINITY FOR THE INTERLEUKIN-10 RECEPTOR, PHARMACEUTICAL COMPOSITION AND USE THEREOF AS IMMUNOMODULATORS FOR TREATING AUTOIMMUNE, INFLAMMATORY OR ALLERGIC DISEASES**

(30) Priority: 05.04.2019 BR 102019007044
(71) Applicant: Universidade Federal de Uberlândia, 38400-902 Uberlândia - MG (BR); Cirino Alberto Goulart Eireli EPP (Biogenetics), 38400-170 Uberlândia - MG (BR)
(72) Inventor: FILHO, Luiz Ricardo Goulart, 38400-652 Uberlãndia (BR); FERREIRA-BRIZA, Fatima D., 5020 Salzburg (AT); VAZ, Emi lia Rezende, 38425-381 Uberlãndia (BR); ARAUJO, Galber Rodrigues, 5020 Salzburg (AT); VIEIRA, Carlos Ueira, 38405-307 Uberlãndia (BR); AGLAS, Lorenz, 5423 Salzburg (AT)
(74) Representative: Bosia, Alessandra
(86) International application number: PCT/BR2020/050116
(87) International publication number: WO 2020/198831

(57) **Abstract**

This invention refers to the selection and characterization of synthetic peptides that have affinity for immune system cells receptors, particularly affinity to the Interleukin-10 (IL-10) receptor, as well as pharmaceutical compositions, and use of these peptides to prepare drugs or immunogenic compounds. These synthetic peptides can bind to cell receptors and promote important regulatory profiles for the treatment and/or prophylaxis of diseases where there is an immune disorder, especially when the diseases related to the immune disorder are: chronic or acute inflammatory diseases, allergic and/or autoimmune.

## Description

### Field of Invention

The present invention relates to the selection and characterization of synthetic peptides, which have an affinity for receptors present in cells of the immune system, particularly with affinity to the Interleukin-10 (IL-10) receptor. These receptors are known to play a significant role in modulating the immune, inflammatory and allergic response. In addition, the present invention also describes pharmaceutical compositions comprising at least one of these synthetic peptides and the use of these peptides to prepare drugs or immunogenic compositions for the treatment or prophylaxis of diseases where there is an immunological disorder, particularly diseases related to immune disorders, such as allergic and/or autoimmune, chronic or acute inflammatory.

### Invention Fundaments

IL-10 is a pleiotropic regulatory cytokine produced by different types of cells: monocytes, T cells, macrophages, dendritic cells (DC), natural killer cells (NK), and B cells. By influencing different cell types, IL-10 is a relevant molecule associated with a wide range of diseases, disorders, and conditions, including inflammatory and allergic conditions, immunity-related disorders, and cancer, due to its ability to regulate the immune response, humoral and cellular. Therefore, understanding how IL-10 regulates different cell types is crucial for developing new intervention strategies in order to treat numerous pathologies.

For example, patent application WO 2008/054585 describes the use of pegylated IL-10 for cancer treatment.

Patent applications WO 2014/172392 and WO 2015/070060 also describes the use of modified, but particularly pegylated, IL-10 to also treat cancer.

It has already been described that IL-10 binds to its respective receptor IL10R, activating the Jak1/Tyk2 proteins. STAT3 is the main protein expressed to down-regulate the inflammatory response, leading to downregulation of some cytokines, such as TNF-α and IL-6. This regulatory process mediated by IL-10 is essential to understand how this cytokine controls the inflammatory response, activating one of the main molecular pathways to fight inflammation and allergic response. On that account, new strategies based on the IL-10 molecule to modulate the production of cytokines have been used in humans to control inflammatory responses.

Recombinant IL-10, for example, has already been shown to be safe, well-tolerated, and effective in patients with Crohn's disease (Fedorak, R. N. et al., Gastroenterology 2000 Dec;119(6):1473-82). Patent application BR 102017010787-6 describes 101 synthetic peptides, selected by Phage Display, which have an affinity for the IL-10 receptor. Said patent application describes the use of these synthesized peptide sequences in the treatment and monitoring of inflammatory processes and allergic and autoimmune diseases.

The Phage Display (PD) methodology may be a suitable option to develop new molecules with pharmaceutical approaches, as it allows us to select small molecules that can mimic specific amino acids from large proteins and induce the same or better response as expected with the natural molecule or in comparison with other recombinant molecules already described. Selected peptides by PD have low toxicity and high receptor/protein affinity. This approach is used to obtain a specific immune response in which the production of IL-10 is essential for the regulation of the inflammatory immune response. Additionally, this type of cell-specific immunomodulation does not lead to severe immunosuppression as seen with commercially available anti-inflammatory drugs.

### Invention Goals

The present patent application aims to select new IL -10 mimetic peptides by PD technology that have an improved ability to modulate the immune response, in particular to control inflammatory and allergic responses. From the studied peptides, four (4) were selected and their three-dimensional structures were predicted. The data obtained showed the ability of these synthesized peptides to reduce the allergic response and inhibit the inflammatory pathways in the tests performed, as well as their pharmaceutical potential in the control of inflammatory or autoimmune allergic diseases.

### Brief Invention Description

The present patent application describes synthetic peptides with affinity for the Interleukin-10 receptor. These peptides were synthesized and their three-dimensional structures were predicted. All peptides were synthesized to have histidine as the first amino acid residue and a sequence of three glycines and one serine as the last four amino acid residues. These peptides showed significant activity on immunological modulatory and regulatory pathways. Therefore, they can be used in the treatment and/or prophylaxis of diseases related to immune disorders, such as chronic or acute inflammatory, allergic, and/or autoimmune diseases.

One modality of the present application is prophylactic and/or treatment methods of diseases related to immune disorders, such as chronic or acute inflammatory, allergic and/or autoimmune diseases, including administering to an individual a pharmaceutically effective dose of at least one of the described synthetic peptides.

Another modality of the present application is pharmaceutical compositions comprising at least one of the synthetic peptides described herein and at least one pharmaceutically acceptable vehicle, carrier and/or compound. The pharmaceutical composition is for the treatment and/or prophylaxis of diseases related to immune disorders such as chronic or acute inflammatory, allergic, and/or autoimmune diseases.

Another modality of the present application is the use of the synthetic peptides or pharmaceutical compositions described herein for the manufacture of a drug or immunogenic composition for use in the treatment and/or prophylaxis of diseases associated with an immunological disorder, such as chronic or acute inflammatory, allergic and/or autoimmune diseases.

### Brief Figures Description

The present invention will be better understood based on the following description below, taken in conjunction with the attached figures, in which
FIGURE 1A shows the predicted three-dimensional structure of Pep 1.
FIGURE 1B shows the predicted three-dimensional structure of Pep 2.
FIGURE 1C shows the predicted three-dimensional structure of Pep 3.
FIGURE 1D shows the predicted three-dimensional structure of Pep 4.
FIGURE 1E shows the aggregation behavior of peptides Pep 1, Pep 2, Pep 3, and Pep 4 in solution.
FIGURE 2A shows an analysis of NFkB expression after 12 hours of treatment with the synthesized peptides described herein.
FIGURE 2B shows an analysis of NFkB expression after 24 hours of treatment with the synthesized peptides described herein.
FIGURE 2C shows an analysis of NFkB expression after 48 hours of treatment with the synthesized peptides described herein.
FIGURE 3A shows an analysis of NFkB expression using an activating molecule, TNF-α, not only in control but after pretreatment with the synthesized peptides for 12 hours.
FIGURE 3B shows an analysis of NFkB expression using an activating molecule, TNF-α, not only in control but after pretreatment with the synthesized peptides for 24 hours.
FIGURE 3C shows an analysis of NFkB expression using an activating molecule, TNF-α, not only in control but after pretreatment with the synthesized peptides for 48 hours.
FIGURE 4A shows an analysis of NFkB expression when the cells were pretreated with TNF-α, later peptides were added after the cells were treated for 12 hours.
FIGURE 4B shows an analysis of NFkB expression when the cells were pretreated with TNF-α and later peptides were added after the cells were treated for 24 hours.
FIGURE 4C shows an analysis of NFkB expression when the cells were pretreated with TNF-α and later peptides were added after the cells were treated for 48 hours.
FIGURE 5A shows an analysis of the IFN signaling pathway after 12 hours of treatment with the synthesized peptides described herein.
FIGURE 5B shows an analysis of the IFN signaling pathway after 24 hours of treatment with the synthesized peptides described herein.
FIGURE 5C shows an analysis of the IFN signaling pathway after 48 hours of treatment with the synthesized peptides described herein.
FIGURE 6A shows an analysis of the IFN signaling pathway when the cells were pretreated with IFN and peptides were later added after the cells were treated for 12 hours.
FIGURE 6B shows an analysis of the IFN signaling pathway when the cells were pretreated with IFN and later peptides were added after the cells were treated for 24 hours.
FIGURE 6C shows an analysis of the IFN signaling pathway when the cells were pretreated with IFN and later peptides were added after the cells were treated for 48 hours.
FIGURE 7A demonstrates the ability of the peptides to reduce TLR4 signaling pathway activation after 12 hours of treatment with the synthesized peptides.
FIGURE 7B demonstrates the ability of the peptides to reduce activation of the TLR4 signaling pathway when cells were pretreated with lipopolysaccharides (LPS). The synthesized peptides were added later, after 12 hours of treatment.
FIGURE 8A shows the effect of the treatment with synthesized peptides in reducing cell activation in CDllc+ CD86 cells after 24 hours of treatment.
FIGURE 8B shows the effect of the treatment with synthesized peptides in reducing cell activation in CDllc+ CD40+ cells after 24 hours of treatment.
FIGURE 9 shows the ability of the synthesized peptide Pep 1 to decrease dendritic cell activation after 24 hours of treatment.
FIGURE 10A shows the effect of synthesized peptide Pep 1 on IgE-mediated release.
FIGURE 10B shows the effect of rIL10 on IgE-mediated release.
FIGURE 11A shows the decrease in IL -6 levels when the cells were treated with Pep 1 followed by incubation with LPS (1 mg/ml) for 24 hours.
FIGURE 11B shows the decrease in MCP-1 levels when the cells were treated with Pep 1 followed by incubation with LPS (1 mg/ml) for 24 hours.
FIGURE 11C shows the decrease in TNF-α levels when the cells were treated with Pep 1 followed by incubation with LPS (1 mg/mL) for 24 hours.

### Detailed Invention Description

The present patent application describes four (4) synthetic peptides with affinity to the Interleukin-10 receptor, comprising or consisting of the following amino acid sequences: Peptide 1 (Pep 1): SEQ ID N° 01; Peptide 2 (Pep 2): SEQ ID N° 02; Peptide 3 (Pep 3) SEQ ID N° 03; Peptide 4 (Pep 4) SEQ ID N° 04. These peptides have significant activity on immunological modulatory and regulatory pathways. Therefore, they can be used in the treatment and/or prophylaxis of diseases related to immune disorders, such as chronic or acute inflammatory, allergic, and/or autoimmune diseases.

The peptides described in this patent application were selected using the Phage Display method (PD). They were obtained randomly after three rounds of selection using a PhD-7mer and PhD12 random peptide library. The selection was performed using the J774 cell line; a competitive elution with rIL-10 (recombinant IL -10) was used to select peptides with affinity for the IL -10 receptor.

A pre-screening was performed on Peripheral Blood Mononuclear Cells (PBMC) using phage supernatant to select phages from the PhD-7mer and PhD12 libraries.

The most reactive phages were purified and tested against PBMC to confirm the ability of selected phages to bind to cell receptors.

Peptides with high affinity for cell receptors were then chemically synthesized using the phage selection manual (Barbas, CF et al. Phage Display: a laboratory manual. Cold Spring Harbor Laboratory Press (2001)), and their three-dimensional structures were predicted. All peptides were synthesized to have Histidine as the first amino acid residue and a sequence of three Glycines and one Serine as the last four amino acid residues.

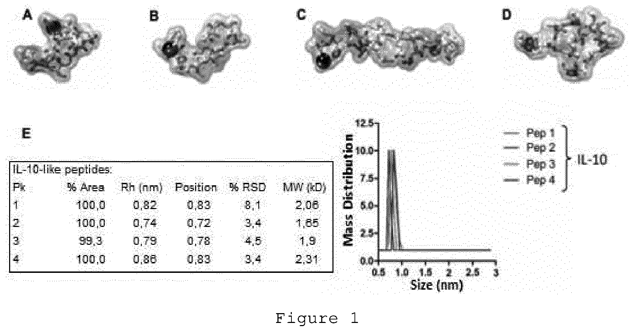

The three-dimensional structure of peptide 1 (Pep 1), SEQ ID N°. 01, with disulfide bridges, was predicted in Figure 1A.

The three-dimensional structure of peptide 2 (Pep 2), SEQ ID N°. 02, without disulfide bridges, was predicted in Figure 1B.

The three-dimensional structure of peptide 3 (Pep 3), SEQ ID N°. 03, was predicted in Figure 1C.

The three-dimensional structure of peptide 4 (Pep 4), SEQ ID N° 04, was predicted in Figure 1D.

Pep 1 and Pep 2 were synthesized with at least 85% identity or similarity between them. The amino acid residues at positions three (3) and eleven (11) were replaced from Serine in Pep 2 to Cysteine in Pep 1. Therefore, Pep 1 is the only peptide synthesized with a disulfide bridge, its three-dimensional structure is different from that of Pep 2 and is more compact compared to Pep 3 and Pep 4. In Figures 1A to 1D, the black sphere represents the C-alpha atom of the first residue, for orientation only, while the hydrophilic residues are represented in blue and the hydrophobic ones in red.

The aggregation behavior of peptides Pep 1, Pep 2, Pep 3, and Pep 4 in solution was determined by Dynamic Light Scattering (DLS), as shown in Figure 1E. All synthetic peptides were approximately 100% monomeric in solution. The measured hydrodynamic radius of the peptides was similar for Pep 1 (0.82nm), Pep 2 (0.74m), Pep 3 (0.79nm), and Pep 4 (0.86nm) .

After the selected peptides were synthesized, several assays were performed to determine their activity. It is noteworthy that in the examples we will show below and in the attached figures, all peptides in general and in different concentrations showed surprising and positive results in modulating the immune, inflammatory and allergic response. Throughout the description, we will highlight by way of example those results that were more statistically significant. This does not exclude other results that were not explicitly mentioned in the examples in the descriptive report; which can be checked directly in the figures.

### Example 1 - Reporter Cells Treatment

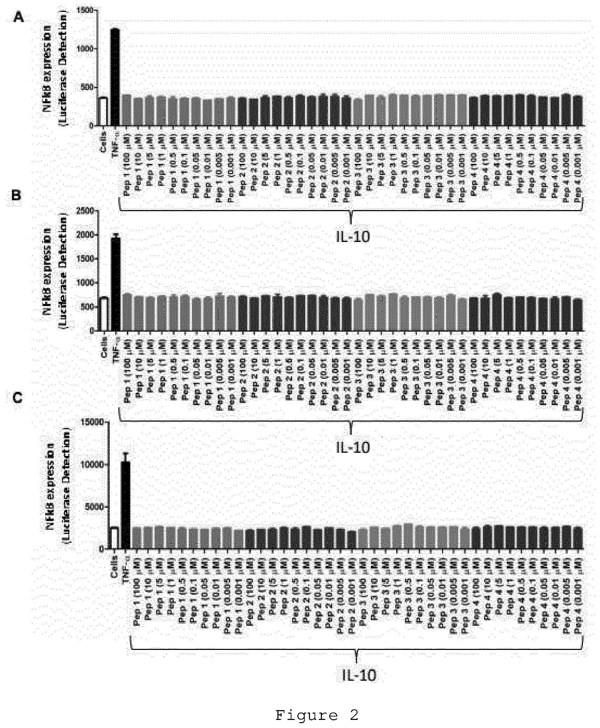

To investigate the ability of the peptides to interfere with the NFkB, IFN, and TLR4 pathways, reporter cell lines were used. To analyze whether the isolated peptides (without activation molecules) could induce any type of activation, the isolated peptides were tested in the cell lines during 12, 24, and 48 hours of treatment. In all reporter cells and at all time points tested, the peptides did not trigger any activation when alone, without any inflammatory environment that would have induced activation of NFKB, IFN, or TLR4 signaling pathways. As an example, Figures 2A, 2B, and 2C show an analysis of NFkB expression after stimulation with the synthesized peptides described in this report. The cells were treated with the synthesized peptides Pep 1, Pep 2, Pep 3, and Pep 4 at various concentrations for 12, 24, and 48 hours, without activating molecules. Luciferase was then measured after 12 hours of treatment (Figure 2A), 24 hours of treatment (Figure 2B), and 48 hours of treatment (Figure 2C). All peptide concentrations tested were unable to elicit any response. TNF-α (200 ng/mL) was used as a positive control.

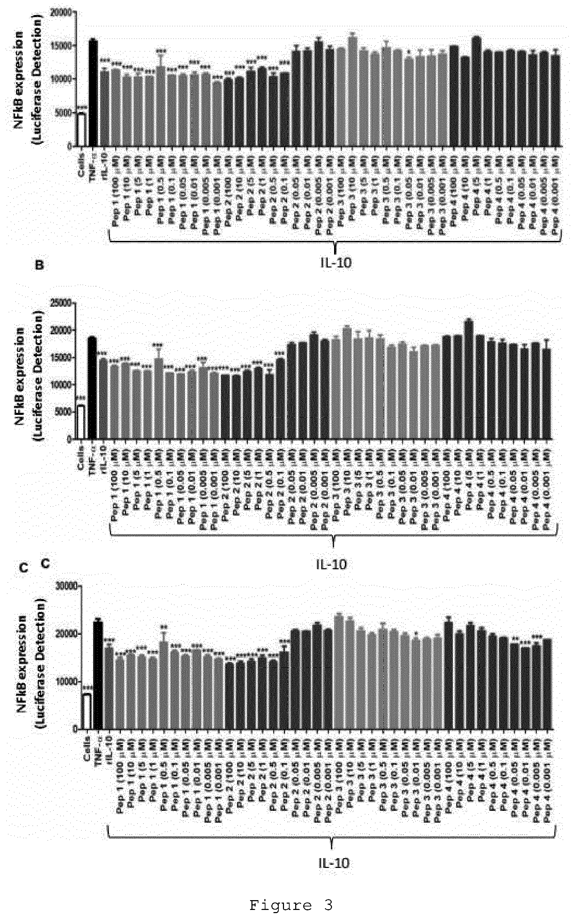

The assay was then performed using TNF-α as the activating molecule not only in the control, but after a pre-treatment with the synthesized peptides Pep 1, Pep 2, Pep 3, and Pep 4 at various concentrations. The pre-treatment with Pep 1 at all concentrations tested was able to decrease NFKB activation at 12, 24, and 48 hours (P <0,0005) of stimulation with TNF-α (Figure 3A, B, and C). The pre-treatment with Pep 2 had the most statistically significant results at 100 µM, 10 µM, 5 µM, 1 µM, 0,5 µM, and 0,1 µM and also decreased NFκB activation (P <0,0005) at 12, 24, and 48 hours (Figure 3A, B, and C). The pre-treatment with Pep 3 had the most statistically significant results at 0,05 µM (P < 0,05) reducing pathway activation after 12 hours (Figure 3A) and at 0,01 µM (P < 0,05) after 48 hours of treatment (Figure 3C). The pre-treatment with Pep 4 produced the most statistically significant results at 0,05 µM, 0,01 µM, and 0,005 µM and reduced pathway activation after 48 hours of treatment (Figure 3C). In the assays shown in Figures 3 A, B, and C, NFkB expression analysis was performed in Jurkart-Dual reporter cells. Cells were treated with peptides for 1 hour and TNF-α (200 ng/ml) was added. Luciferase was measured after 12 hours of treatment (Figure 3A), 24 hours of treatment (Figure 3B), and 48 hours of treatment (Figure 3C) .

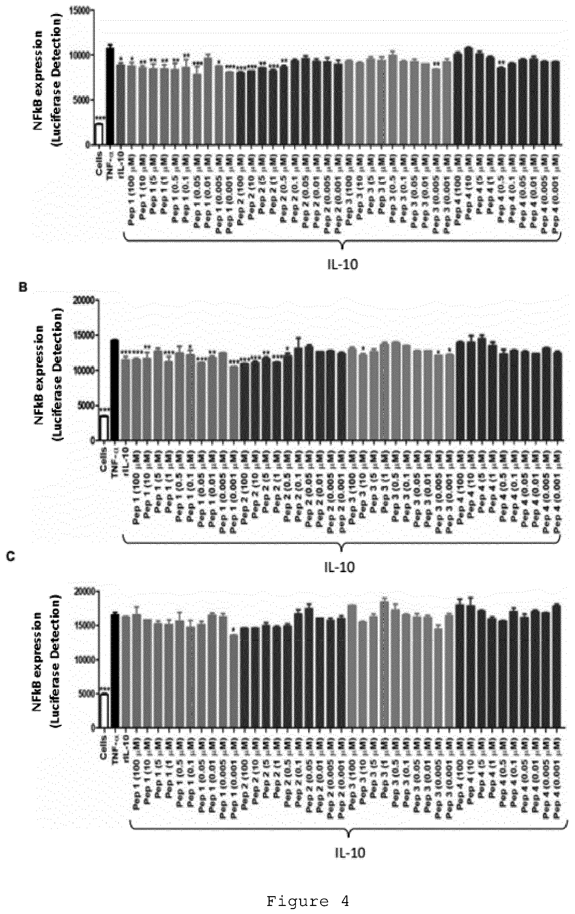

Then another assay was performed in which the cells were pretreated with TNF-α and the peptides were added later. For example, the results with the greatest statistical significance were: Pep 1 at concentrations of 10 µM, 5 µM, 1 µM, 0,5 µM, 0,1 µM (P < 0,05); 0,05 µM and 0,001 µM (P < 0,0005) decreased the activation of the analyzed pathway after 12 hours of treatment (Figure 4A); Pep 2 at concentrations of 100 µM (P < 0,0005), 10 µM (P < 0,005), 5 µM (P < 0,05) and 1 µM (P < 0,005); Pep 3 at a concentration of 0,005 µM (P < 0,005) and Pep 4 at a concentration of 0,5 µM (P < 0,05) were also able to decrease NFκB signaling after 12 hours of treatment (Figure 4A). After 24 hours of treatment with the peptides, the results with the greatest statistical significance were: Pep 1 at 100 µM (P < 0,0005), 10 µM (P < 0,005), 1 µM (P < 0,0005), 0,1 µM (P < 0,05), 0,005 µM (P < 0,0005), 0,001 µM (P < 0,005), and 0,0001 µM (P < 0,0005); Pep 2 at 100 µM (P < 0,0005), 5 µM (P < 0,005), 1 µM (0,0005), and 0,5 µM (P < 0,05); and Pep 3 at 10 µM (P < 0,05), 0,005 µM (P < 0,05), and 0,001 µM (P < 0,05), with less activation of the analyzed signaling pathway (Figure 4B). After 48 hours of treatment, Pep 1 at a concentration of 0,001 µM (P < 0,05) was the one that produced a result with the highest statistical significance in reducing NFκB signaling (Figure 4C). In the assays shown in Figures 4 A, B, and C the analysis of NFkB expression was performed in Jurkart-Dual reporter cells. Cells were treated with TNF-α (200 ng/ml) for 1 hour and the peptides were added. Luciferase was measured after 12 hours of treatment (Figure 4A), 24 hours of treatment (Figure 4B), and 48 hours of treatment (Figure 4C). TNF-α (200ng/mL ) was used as a positive control and rIL-10 ( Recombinant Interleukin-10) (0,4ng/mL ) as a negative control. * P <0,05; ** P <0,005; *** P <0,0005.

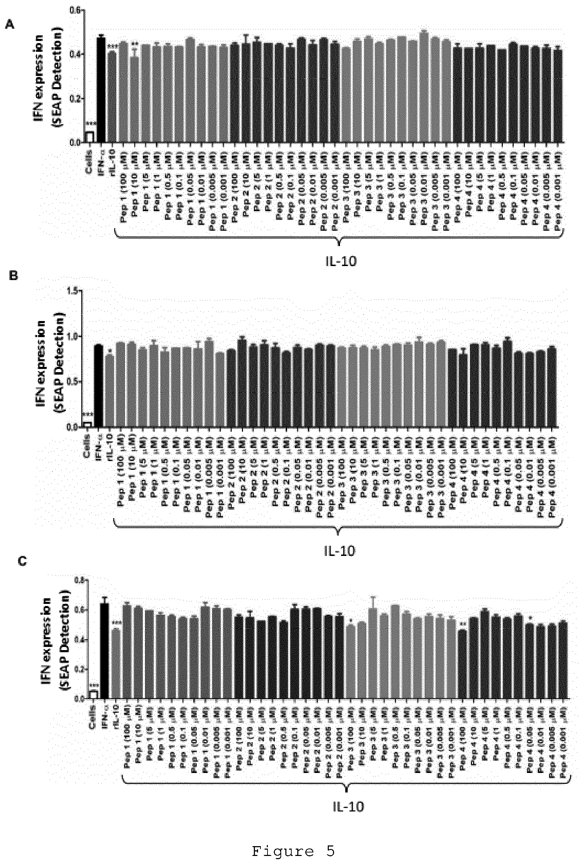

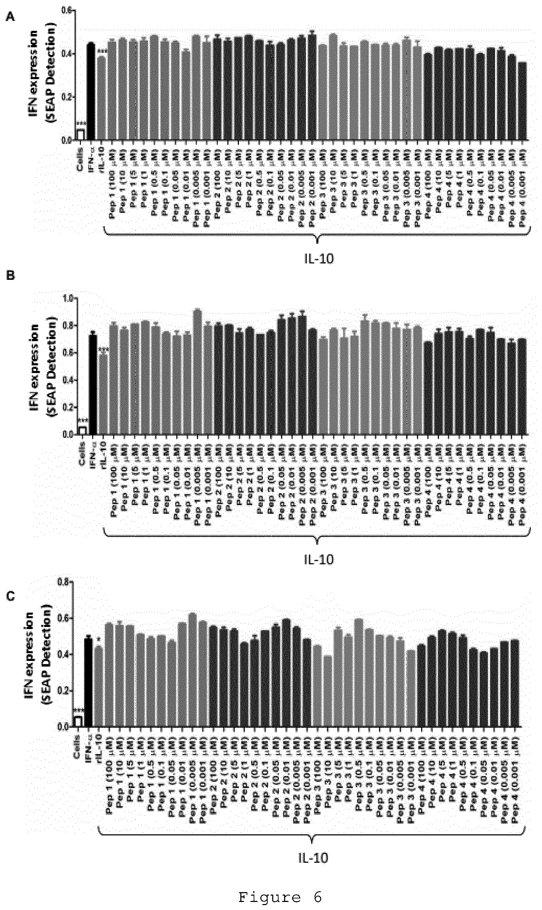

Analysis of the IFN signaling pathway showed that activation of the pathway was reduced when Jurkat cells were pretreated with Pep 1 at a concentration of 10 µM (P < 0,005) for 12 hours (Figure 5A); Pep 3 at a concentration of 100 µM (0,005) for 48 hours (Figure 5C); and Pep 4 at a concentration of 100 µM (P < 0,005) and 0,05 µM (P < 0,05) for 48 hours (Figure 5C). When the cells were pretreated with IFN, although the peptides showed results at some concentrations, they were statistically insufficient. Therefore, we cannot confirm whether they were able to affect the activation pathway (Figure 6A, B, and C) in the same or better way than the negative control (rIL-10). In the assays shown in Figures 5 (A, B, and C) and 6 (A, B, and C), IFN expression was analyzed in Jurkart-Dual reporter cells. In the assays shown in Figures 5 A, B, and C, cells were treated with peptides for 1 hour followed by the addition of IFN-α (104 EU/ml). Secreted Embryonic Alkaline Phosphatase (SEAP) production was measured after 12 hours of treatment (Figure 5A), 24 hours of treatment (Figure 5B), and 48 hours of treatment (Figure 5C). In the assays shown in Figures 6 A, B, and C, cells were treated with IFN-α (104 EU/ml) for 1 hour, and then peptides were added. SEAP production was measured after (D) 12 hours of treatment (E) 24 hours of treatment, and (F) 48 hours of treatment. IFN-α (104 EU/mL) was used as a positive control and rIL-10 (0,4 ng/mL) as a negative control. * P <0,05.

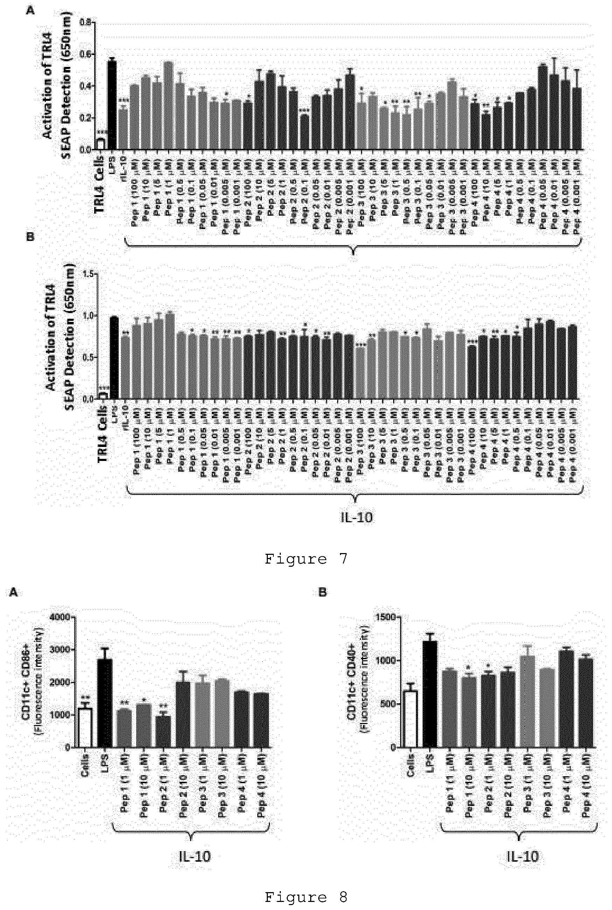

HEK cells were used to investigate the ability of the peptides to reduce activation of the TLR4 signaling pathway after 12 hours of treatment. Pre-treatment with the peptides showed that Pep 1 at 0,005 µM (P <0,05); Pep 2 at 100 µM (P <0,05), 0,1 µM (P <0,0005); Pep 3 at 100 µM (P <0,05) and Pep 4 at 100 µM (P <0,05), 10 µM (P <0,005), 5 µM (p <0,05), and 1 µM (P <0,05) significantly reduced pathway activation (Figure 7A). In contrast, when cells were pretreated with lipopolysaccharides (LPS) and peptides were added, activation of the TLR4 pathway was more significantly reduced with addition of Pep 1 at 0,1 µM (P <0,05), 0,05 µM (P <0,05), 0,01 µM (P <0,005), 0,005 µM (P <0,005) and 0,001 µM (P <0,005); Pep2 at 100 µM (P <0,05), 1 µM (P <0,005), 0,5 µM (P <0,05), 0,1 µM (P <0,05), 0,05 µM (P <0,05) and 0,01 µM (P <0,005); Pep 3 at 100 µM (P <0,0005), 10 µM (P <0,005), 0,5 µM (P <0,05) and 0,1 µM (P <0,05); Pep 4 at 100 µM (P <0,0005), 10 µM (P <0,05), 5 µM (P <0,005), 1 µM (P <0,05) and 0,5 µM (P <0,005) (Figure 7B).

In the assay shown in Figure 7A, the cells were treated with peptides for 1 hour, then LPS (100ng/ml) was added. SEAP production was measured after 12 hours of treatment. In the assay shown in Figure 7B, the cells were treated with LPS (100ng/ml) for 1 hour, then the peptides were added. SEAP production was measured after 12 hours of treatment. LPS (100 ng/mL) was used as positive control and IL -10 (0,4ng/mL) as negative control. * P <0,05; ** P <0,005; *** P <0,0005.

### Example 2 - Peptides effect on dendritic and T cells in an inflammatory and allergic environment

Dendritic cells from mice bone marrow were isolated to analyze the effect of synthesized peptides Pep 1, Pep 2, Pep 3, and Pep 4 at two concentrations, 1 µM, and 10 µM, on reducing cell activation. After 24 hours of treatment with a composition comprising at least one of the peptides at the desired concentration, cell analysis showed that all four synthesized peptides showed responses at different levels and were able to affect the co-stimulatory molecules present on the surface of the dendritic cells, with peptides Pep 1 and 2 showing the best response. Thus, at 1 µM, Pep 1 decreased the expression of CD86+ (P < 0,005), and at 10 µM it also decreased the expression of CD86+ (P < 0,05). Pep 2 at a dosage of 1 µM decreased the expression of CD86+ and CD40+ on the cell surface (P < 0,005; P < 0,05, respectively) (Figure 8 A and B).

In the assays, CDllc+ CD86 cells (Figures 8A) and CDllc+ CD40+ cells (Figures 8B) were used after 24 hours of treatment. LPS (100ng/mL) was used as a positive control. * P <0,05; ** P <0,005.

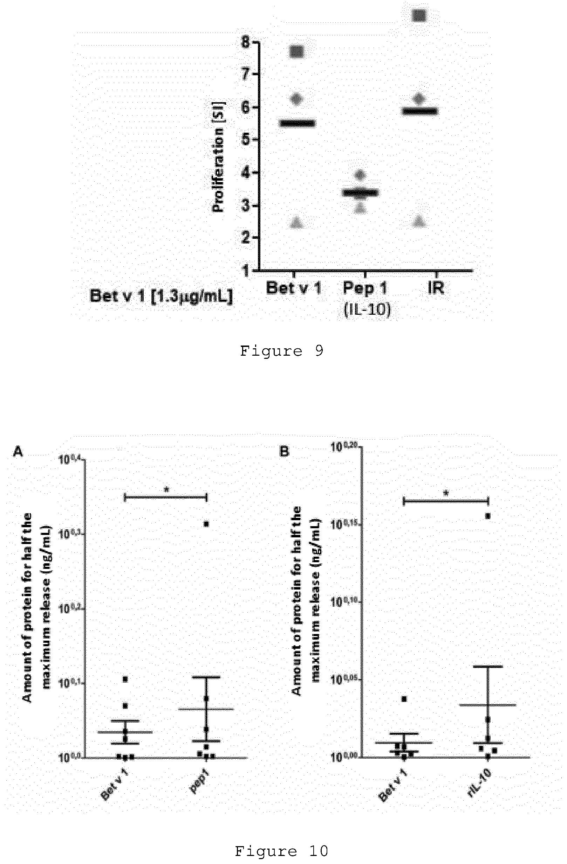

To confirm the ability of the synthesized peptides to decrease dendritic cell activation, a composition comprising Pep 1 at a concentration of 10 µM was chosen and the proliferation of T cells from allergic patients was measured after 24 hours of treatment. Pep 1 at this concentration decreased T cell proliferation (P < 0.005) (Figure 9). Cells were treated with Bet v 1 as a positive control, and an irrelevant peptide (IR) was used as a negative control.

All four synthesized peptides showed an effect on immune cells in inflammatory and allergic environments, confirming their ability to modulate the immune response. Moreover, the results presented here demonstrate the potential for an association of these peptides, since they showed different responses and, in several cases, complementary in terms of concentration and exposure time with a better response. Therefore, it is clear that they can be used alone or in any combination, in compositions for the preparation of drugs or immunogenic compositions for the treatment and/or prophylaxis of diseases in which an immunological disorder is present, especially in which disorders associated with the immunological disorders are: chronic or acute inflammatory, allergic and/or autoimmune diseases.

Since all peptides showed a response, we selected Pep 1 to further extend the studies. These results can be extrapolated to other synthetic peptides with affinity for the IL -10 receptor synthesized and demonstrated in the present patent application.

### Example 3 - Pep 1 action on basophil degranulation

The effect of Pep 1 on IgE-mediated release was studied using mouse basophilic leukemia cells transfected with the human FcεRI IgE receptor (hRBL). The hRBL cells were sensitized with serum from seven patients allergic to birch pollen. The cells were stimulated with the antigen and its interaction with immobilized IgEs resulting in the release of β-hexosaminidase.

When cells were treated with a composition comprising Pep 1 to 1 µM (Figure 10A) or rIL10 (Figure 10B), the amount of Bet v 1 required to induce the same amount of degranulation was higher than for cells treated with Bet v1 alone (P < 0,05). Amount of rBet v 1 (ng/mL) required to induce half the release of β-hexosaminidase using serum from seven birch pollen-allergic donors after treatment with Pep 1 (A) (P < 0,05) and rIL10 (B ) * P < 0,05.

### Example 4 - Cytokines production after treatment of cell line J774 with Pep 1

After 24 hours of treatment, the produced amounts of IL -6, MCP-1, and TNF-α were measured. No interference in IL -10, IFN-γ, or IL12p70 production was observed (data not shown). Cells treated with the synthetic peptide Pep 1 alone, without LPS stimulation to induce an inflammatory response, were unable to elicit any response (data not shown). In contrast, when cells were treated with a composition of Pep 1 followed by incubation with LPS (1 mg/ml) for 24 hours to induce an inflammatory response, the peptide was able to decrease the production of IL -6, MCP -1, and TNF-α.

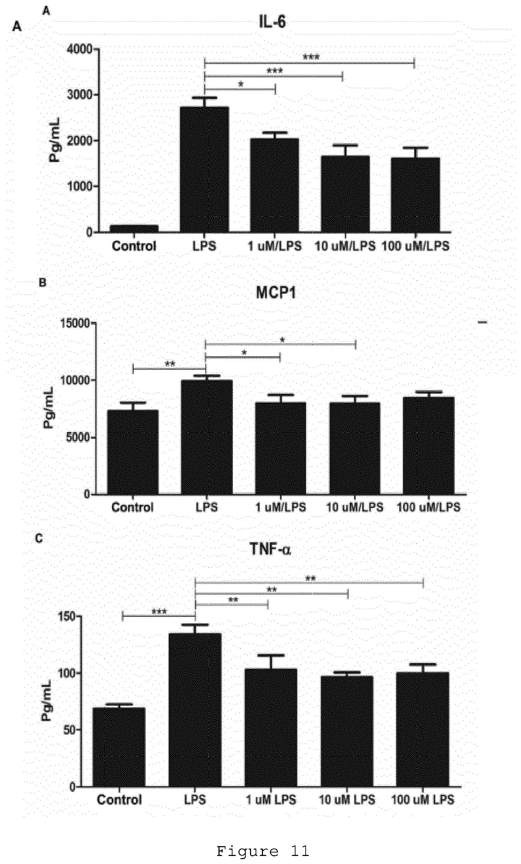

Pep1 (1 µM, 10 µM and 100 µM, 1 µM P < 0,05 and 10 µM and 100 µM P < 0,0005) significantly reduced the levels of IL -6 (1 µM and 10 µM, P < 0,05) (Figure 11A); MCP-1 (Figure 11B) and TNF-α (1 µM, 10 µM and 100 µM, P < 0,005) (Figure 11C). Each decrease was significant compared to cells treated with LPS alone (positive control). * P <0,05; ** P <0,005; *** P <0,0005

Results analysis presented in the examples and embodiments of the invention

To analyze whether the structure is crucial for binding activation and cellular response, or whether sequence alone is sufficient to trigger this effect, we chose to synthesize a single conformational peptide, Pep 1, with a disulfide bridge and at least 85% identity or similarity to Pep 2, and the others with a linear chain (without a disulfide bridge). The structures of all 4 peptides were predicted to investigate their conformational differences. Pep 1 had a more compact structure compared to peptides 2, 3, and 4 (Figure 1 A, B, C, and D). All synthetic peptides showed a response, although compact peptides were better at inducing a response because conformation is essential for receptor activation. All synthetic peptides are present as monomers, as shown by the DLS results (Figure 1E).

The cytokine IL -10 binds to IL -10R, activating JAK /STAT20 signaling; this signaling pathway is the main mechanism to produce cytokines that can control an inflammatory response. However, different cytokines can activate this pathway, so their activation depends on which JAK and STAT family is activated and thus is or is not, responsible for triggering the anti-inflammatory effect. The production of SOCS 3 is mediated by IL -10R and can interfere with the release of IκB from the NFκB complex in intact cells and/or block the binding of NF-KB DNA already present in the nucleus and consequently block the expression of NFκB. SOCS 3 production may also inhibit LPS signaling and activate the TLR4 pathway, although this mechanism is complex and not fully elucidated. To investigate the ability of the peptides to block the activation of inflammatory pathways, reporter cells were treated with the peptides before or after the addition of activating molecules (TNF-α, IFN-α, and LPS) to explore new treatment strategies.

After pretreatment with synthetic peptides, Pep 1 showed a better ability to interfere with the metabolic pathways analyzed compared to Pep 2, Pep 3, and Pep 4. At all concentrations tested and at all time points, Pep 1 was able to reduce NFKB expression; this response was maintained over 48 hours of treatment. Pep 2 also interfered with these pathways after 48 hours, but with a greater significance only at some of the concentrations tested (100 µM, 10 µM, 5 µM, 1 µM, 0,5 µM, and 0,1 µM). The same effect did not occur as significantly when cells were treated with Pep 3 or Pep 4. Thus, we concluded that although these two peptides elicited a response and therefore have the potential for use, this response was not as stable as observed when treated with Pep 1 and 2. At the same time, we confirmed the best effect of Pep 1 when cells were pretreated with TNF-α (to activate the NFκB pathway), even when the cells were already activated; all concentrations tested decreased NFκB activation after 12 and 24 hours. A similar effect was seen in the TLR4 pathway, where Pep 1 (at different concentrations) was stable when the pathway was activated before treatment with the peptide. This improved effect observed after treatment with Pep 1 can be explained by the disulfide bridge present in the peptide, which leads to a better conformation required for the activation of the receptor and the reduction of the signaling pathways studied. On the other hand, this difference in the synthesized peptides action indicates the possibility of using them in combination, be it any combination of two or more of them, to obtain a synergistic, improved, and/or long-term result, depending on the objective, since they showed a different effect depending on the concentration and the duration of exposure.

When Jurkat cells were used to study the ability of the peptides to inhibit the IFN-α pathway, the peptides caused a statistical decrease at certain concentrations. This response may not be stable because although IFN-αβ also induces signaling JAK-STAT, activation is preferentially triggered by STAT4, 1, and 2 rather than STAT3, as occurs after IL -10 binds to IL -10R. Moreover, rIL-10 can also induce IFN production, suggesting that the cytokine can interact with both signaling pathways.

In studies on the mechanism of immune tolerance to allergens, research has shown the role of IL -10 in reducing the expression of co-stimulatory molecules in antigen-presenting cells (APCs), and consequently, T cells are not activated and do not proliferate, suppressing the exacerbation of inflammation, which is considered a critical pathway for preventing a severe allergic inflammatory response. This idea is corroborated by the fact that the synthesized peptides reduced the co-stimulatory molecules in dendritic cells (Figure 8 A and B), indicating a stable capacity and effect. Although all the synthesized peptides showed results with potential for use, especially Pep 1 and 2, we selected Pep 1 for some experiments to investigate its regulatory effect, an effect that was confirmed by the decrease in T cell proliferation in allergic patients (Figure 9). However, TLR4 was shown to be present in dendritic cells, since Pep 1 and 2 statistically decreased this activation pathway in reporter cells, as well as co-stimulatory molecules in dendritic cells. We can accept the hypothesis that the peptides synthesized here may act in both cases: directly in the TLR4 pathway to reduce the production of pro-inflammatory cytokines, and also to reduce antigen presentation to T cells and consequently decrease their proliferation.

Another effect of IL -10 in the allergic response is the inhibition of the high-affinity receptor for IgE (FcεRI) in human and murine mast cells, reducing the release of β-hexosaminidase. In addition, the cytokine interferes with regulatory molecules (Fyn, Syk, Akt, and STAT5) found in activated mast cells. Since mast cells and basophils have similar crosstalk between FcεRI and IgE31, we can assume that IL -10 has the same effect in decreasing FcεRI expression in basophils as in mast cells, which may explain how Pep1 can decrease the observed basophil degranulation in the RBL assay (Figure 10).

Macrophages are relevant cells in inflammatory responses. Treatment with LPS induces the transcription of NF-KB and MAPK32 and subsequently the production of IL -6, MCP-1, and TNF-α33. However, the treatment of macrophages with the IL -10 molecule, in an inflammatory environment, can negatively regulate the production of TNF-α, MCP-1, and IL -634. This finding suggests that the peptides synthesized here have similar activity to the native molecule since treatment with Pep 1 decreased these cytokines after treating the J447 cell line with LPS (Figure 11). Production of MPC-1 and IL -6 in an inflammatory environment allows the activation of monocytes, memory T lymphocytes, natural killer (NK) cells, and macrophages. Then, a new strategy to block or reduce these cytokines should be an interesting treatment against a specific target. Therefore, the peptides Pep 1, 2, 3, and 4 synthesized herein can be used alone or in any combination with each other in the treatment of diseases related to immune disorders, such as chronic or acute inflammatory, allergic and/or autoimmune diseases, since these peptides can modulate NFκB and TLR4 signaling, dendritic cell activation, T cell proliferation, and basophil degranulation.

Pep 1 showed a better ability to interact with cell receptors on reporter cells, BMDCs (murine bone marrow dendritic cells), basophils, T cells, and cell line J774, and was shown to regulate an allergic inflammatory response better than Pep 2, Pep 3, and Pep 4 in comparison. However, all these synthetic peptides showed the ability to modulate an allergic inflammatory response at different levels, and they can be used in the development of pharmaceutical compositions, alone or in combination with other peptides or compounds, to develop new treatments or prevent inflammatory and/or allergic diseases.

Therefore, the present patent application describes synthetic peptides with affinity for the Interleukin-10 receptor comprising or consisting of one of the amino acid sequences selected from the group consisting of: SEQ ID N° 01, SEQ ID N° 02, SEQ ID N° 03, and SEQ ID N° 04, or a sequence having at least 85% identity or similarity to one of the amino acid sequences selected from the group consisting of: SEQ ID N° 01, SEQ ID N° 02, SEQ ID N° 03, and SEQ ID N° 04. The peptide comprising or consisting of the sequence SEQ ID N° 01 exhibits a three-dimensional conformation with disulfide bridges. The peptide comprising or consisting of the sequence SEQ ID N° 02, SEQ ID N° 03, or SEQ ID N° 04 has a three-dimensional conformation free of disulfide bridges. Synthetic peptides are intended to be used in pharmaceutical compositions in the treatment and/or prophylaxis of diseases associated with an immune disorder, wherein diseases associated with an immune disorder are chronic or acute inflammatory, allergic, and/or autoimmune diseases.

The present application also describes methods for the treatment and/or prophylaxis of diseases associated with an immunological disorder, comprising administering to an individual a pharmaceutically effective dose of at least one of the synthetic peptides described, or administering to an individual a pharmaceutical or immunogenic composition comprising at least one of the synthetic peptides described.

The present application also describes pharmaceutical or immunogenic compositions comprising at least one of the synthetic peptides described herein and at least one pharmaceutically acceptable carrier or compound. The pharmaceutical or immunogenic composition is for use in the treatment and/or prophylaxis of diseases associated with an immune disorder, wherein the diseases associated with an immune disorder are chronic or acute inflammatory, allergic, and/or autoimmune diseases.

In addition, the present application also describes the use of the synthetic peptides or pharmaceutical compositions described herein for the manufacture of a drug or immunogenic composition for use in the treatment and/or prophylaxis of diseases associated with immunological disorders.

Although in this patent application the subject matter of the present invention has been described in some detail with illustrations and examples for purposes of clarity and understanding, it will be apparent that certain changes and modifications may be made within the scope of the appended claims.

The examples described herein are not limiting and allow the person skilled in the art to modify some aspects or components of the present invention to correspond to the synthetic peptides, compositions, or uses described herein without departing from the scope of the present invention.

## Claims

1. Synthetic peptide with affinity to the Interleukin-10 receptor **characterized by** the fact that it comprises one of the amino acid sequences selected from the group consisting of: SEQ ID N° 01, SEQ ID N° 02, SEQ ID N° 03, and SEQ ID N° 04, or a sequence having at least 85% identity or similarity to one of the amino acid sequences selected from the group consisting of: SEQ ID N° 01, SEQ ID N° 02, SEQ ID N° 03 and SEQ ID N° 04.

2. Synthetic peptide according to claim 1, **characterized by** the fact that the peptide comprising SEQ ID N° 01 has a three-dimensional formation with disulfide bridges.

3. Synthetic peptide according to claim 1, **characterized by** the fact that the peptide comprising SEQ ID N° 02, SEQ ID N° 03, or SEQ ID N° 04 has a three-dimensional conformation free of disulfide bridges.

4. Synthetic peptide according to any one of claims 1 to 3, **characterized by** the fact that it is to be applied in pharmaceutical compositions for use in the treatment or prophylaxis of diseases related to immune disorder.

5. Synthetic peptide according to claim 4, **is characterized by** the fact that the diseases related to immune disorder are: chronic or acute inflammatory diseases, allergic and/or autoimmune.

6. Pharmaceutical composition **characterized by** the fact that it comprises at least one of the synthetic peptides as described in any one of claims 1 to 5 and at least one pharmaceutically acceptable vehicle, carrier, or compound.

7. Pharmaceutical composition according to claim 6, **is characterized by** the fact that it is for use in the treatment and/or prophylaxis of diseases related to immune disorder.

8. Pharmaceutical composition according to claim 7, **is characterized by** the fact that the diseases related to the immune disorder are: chronic or acute inflammatory diseases, allergic, and/or autoimmune.

9. Use of at least one synthetic peptide as described in any one of claims 1 to 5 or of the pharmaceutical composition as defined in claim 6, **characterized by** the fact that it is for the preparation of a drug or immunogenic composition for use in treatment or prophylaxis of diseases related to immune disorder.

10. Use according to claim 9, **characterized by** the fact that the diseases related to immune disorder are: chronic or acute inflammatory diseases, allergic and/or autoimmune.

11. Prophylactic and/or treatment method of diseases related to the immunological disorder, **characterized by** administering to an individual a pharmaceutically effective dose of at least one of the recombinant peptides as described in any one of claims 1 to 5 or a pharmaceutical composition as defined in claim 6.


List os Sequences

<110>  Universidade Federal de Uberlandia - UFU
       Cirino Alberto Goulart EIRELI EPP (BioGenetics)

<120>  SYNTHETIC PEPTIDES WITH INTERLEUKIN-10 RECEPTOR AFFINITY,
PHARMACEUTICAL COMPOSITION AND THEIR USE AS IMMUNOMODULATORS IN
THE TREATMENT OF AUTOIMMUNE, INFLAMMATORY OR ALLERGIC DISEASES

<160>  4

<210>  1
<211>  15
<212>  PRT
<213>  Artificial Sequence

<220>
<223>  INTERLEUKIN-10 MIMETIC PEPTIDE

<400>  1

His Ala Cys Ser Ala Val Ile Lys Ser Ser Cys Gly Gly Gly Ser
1               5                   10                  15

<210>  2
<211>  15
<212>  PRT
<213>  Artificial Sequence

<220>
<223>  INTERLEUKIN-10 MIMETIC PEPTIDE

<400>  2

His Ala Ser Ser Ala Val Ile Lys Ser Ser Ser Gly Gly Gly Ser
1               5                   10                  15

<210>  3
<211>  18
<212>  PRT
<213>  Artificial Sequence

<220>
<223>  INTERLEUKIN-10 MIMETIC PEPTIDE

<400>  3

His Cys Phe Asp Thr Gln Arg Met Glu Phe Arg Met Pro Pro Gly Gly
1               5                   10                  15

Gly Ser

<210>  4
<211>  18
<212>  PRT
<213>  Artificial Sequence

<220>
<223>  INTERLEUKIN-10 MIMETIC PEPTIDE

<400>  4

His Cys Met Thr Gly Ser Arg Ala Thr Phe Pro Ser Ala Pro Gly Gly
1               5                   10                  15

Gly Ser